(19) Europäisches Patentamt / European Patent Office / Office européen des brevets

(11) **EP 3 776 503 B1**

(12) **EUROPEAN PATENT SPECIFICATION**

| | |
|---|---|
| (45) Date of publication and mention of the grant of the patent:<br>**14.12.2022 Bulletin 2022/50** | (51) International Patent Classification (IPC):<br>**G08B 13/00** (2006.01)   **G01N 33/28** (2006.01)<br>**B60S 5/02** (2006.01)   **B67D 7/04** (2010.01)<br>**B67D 7/42** (2010.01) |
| (21) Application number: **19815001.3** | (52) Cooperative Patent Classification (CPC):<br>**B67D 7/04; B67D 7/342; G06Q 20/145;**<br>**G07F 13/025;** G01N 33/28 |
| (22) Date of filing: **01.04.2019** | |
| | (86) International application number:<br>**PCT/TR2019/000027** |
| | (87) International publication number:<br>**WO 2019/236032 (12.12.2019 Gazette 2019/50)** |

(54) **FUEL TYPE IDENTIFICATION AND TRANSFER METHOD AND APPARATUS THEREFOR**

VERFAHREN UND VORRICHTUNG ZUR IDENTIFIZIERUNG DES KRAFTSTOFFTYPS UND ÜBERTRAGUNG

PROCÉDÉ D'IDENTIFICATION ET DE TRANSFERT DE TYPE DE CARBURANT ET APPAREIL CORRESPONDANT

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR** | (73) Proprietor: **Asis Otomasyon Ve Akaryakit Sistemleri Anonim Sirketi**<br>**34775 Ümraniye/Istanbul (TR)** |
| (30) Priority: **13.04.2018 TR 201805287** | (72) Inventor: **KAYA, Yusuf**<br>**34775 Ümraniye/Istanbul (TR)** |
| (43) Date of publication of application:<br>**17.02.2021 Bulletin 2021/07** | (56) References cited:<br>EP-A1- 0 566 345    WO-A1-98/20342<br>DE-A1-102011 112 417    JP-A- H04 352 697<br>US-A1- 2003 209 280    US-A1- 2003 209 280<br>US-A1- 2014 130 939    US-B1- 6 341 629 |
| (60) Divisional application:<br>**22020144.6 / 4 047 365** | |

## Description

### TECHNICAL FIELD

The present invention relates to a control method and apparatus for identifying a fuel type such as gasoline or diesel fuel, and providing that the transfer thereof from a first location to a second location involves the same type of fuel.

### BACKGROUND OF THE INVENTION

Current data estimates that there are globally about 1.2 billion motor vehicles using fossil fuel. This number brings about hundred thousands of active fuel filling and refueling stations. This increasing number and the variety in fuel types require meticulous management of transferring fuel from one location to another. Indeed, engines of vehicles such as land vehicles working on fossil fuel are designed to use a certain type of fuel. It is known that an engine not supplied with the suitable type of fuel would break down, therefore various solutions have been suggested over the years against "wrong fuel feeding" or "cross filling."

For example, US 4838323A describes that the control between two types of fuel such as *gasoline and diesel fuel may be realized by a sensor system that can detect the difference* in vapor pressure between them and a fuel cut-off apparatus that can communicate with such system. According to US4838323A, said sensor system can make detection by sampling the fuel inside the fuel tank of an automobile.

US 6341629A discloses a method for controlling the movement of *a liquid from a first location to a second location via a dispenser for the liquid* located in a liquid line between the first and second location wherein the dispenser includes a nozzle, an internal liquid conduit, a valve, a body portion, a hollow annular collar and a first detector. The first detector is located in the collar which is positioned around the nozzle or between the nozzle and the body portion such that the first detector is in vapor communication with a vapor space above the second location. The vapor from the vapor space is analyzed by withdrawing the vapor past the first detector and using the results of the analysis to control the operation of the valve, thereby determining as to whether fuel transfer will be made or not.

US 5209275A relates to a fuel dispensing apparatus which includes a detecting element for detecting the type of fuel from the gas vaporized from such fuel remaining in a storage tank to be supplied with fuel.

On the other hand, wrong fuel transfer can occur not only between a fuel dispenser and an automobile but also during the refueling of underground tanks at gas stations which can cause extremely inconvenient consequences such as total cleaning of the underground tank. It is also known that other solutions aiming to detect the type of the fuel in liquid form have been generated for such circumstances. However, these solutions have not sufficiently extended through the marked due to the high costs they entail.

An apparatus according to the preamble of claim 7 is known for example from DE 10 2011 112417 A1 or from EP 0566 345 A1.

### SUMMARY OF THE INVENTION

The object of the invention is to effectively identify of a fuel type.

Further object of the invention is to provide effective prevention of faulty fuel transfer from a first location to a second location.

In line with these objects, the present invention relates to a method for controlling the transfer of a known-type fuel from a first location to a second location with an unknown type, comprising the following steps of:

    a. determining the fuel type in the second location,
    b. comparing the fuel type in the second location with the fuel type in the first location,
    c. permitting fuel transfer, in case the fuel type in the first location matches with the fuel type in the second location, and preventing the transfer otherwise,

wherein the determination in step a comprises measuring the density and the *temperature*, or measuring the density and the *pressure of a gaseous form of the fuel in the second location*, and comparing the measured values with a data set comprising predetermined reference values or making a decision through a mathematical modeling.

According to an embodiment of the invention, the type of the fuel in the first location may not be previously known but the type of the fuel in the second location may be previously known. In this case, the type of the fuel *in* the first location may be determined as explained above and the control of the fuel transfer may be made accordingly. According to an embodiment of the invention, the method of the invention may be adapted to a fuel vapor recovery system or such system may involve the method of the invention.

*According to an embodiment of the invention, the fuel types in the first and second* location may not be previously known. In this case, the type of the fuel in both locations may be determined as explained above and the control of the fuel transfer may be made accordingly.

According to an embodiment of the invention, the density measurement of the fuel vapor and the temperature and/or pressure measurement may be made in an environment where the gaseous form is open to the atmosphere.

According to an embodiment of the invention, the gaseous form will not be subject to any pre-treatment before the measurement of density and temperature and/or pressure, such as heating or pressurization.

According to an embodiment of the invention, the first location is a fuel dispenser whereas the second location is the fuel tank of a land vehicle. According to another embodiment of the invention, the first location is the tank of a fuel truck whereas the second location is the (underground) tank of a gas station.

According to another embodiment of the invention, it relates to an apparatus for controlling the transfer of a specific type of fuel from a first location to a second location, comprising a sensor device for detecting the fuel type in one of said locations and a controller communicating with said sensor device, characterized in that the sensor device comprises a gas sensor that can detect the density of the gaseous form of the fuel in said location and at least one gaseous state sensor that can detect the temperature and/or pressure of the gaseous form of the fuel in said location, and that said controller is configured to compare the data received from said sensor device with a data set comprising predetermined reference values or to conduct a mathematical modeling.

The mathematical modeling to be used may be developed on the basis of an equation known in the art, for example the Antoine equation as below:

$$\log P = A - \frac{B}{C+T} \qquad \text{formula I}$$

wherein P is *pressure,* T is temperature, A, B and C are constants pertaining to the component.

In an exemplary embodiment, following the measurement of ambient temperature, the partial pressure of the fuel vapor can be calculated according to the Antoine equation. Since ambient pressure is also a measurable value, the molar fraction of the fuel vapor can be calculated by dividing the determined partial pressure by ambient pressure according to the Antoine equation.

$$y_i = p_i / P \qquad \text{formula II}$$

wherein $y_i$ is molar fraction; pi is partial pressure; and P is ambient pressure.

On the other hand, the total mass of the volatile fuel (vapor) may be obtained based on 1 mole of mixture by multiplying the molar fraction by the total molar weight.

Based on an ideal gas concept, the volume of 1 mole of gas mixture can be calculated by the following equation

$$V = RT / P \qquad \text{formula III}$$

using ambient temperature and pressure conditions.

The total volatile organic compounds mass in unit volume will reveal the VOC value. This calculated value is specific to gasoline or diesel, thus the fuel type which is close to the result read by VOC may be determined.

According to an embodiment of the invention, it relates to an apparatus for controlling the transfer of a specific type of fuel from a first location to a second location comprising a sensor device for detecting the fuel type in a location and a controller communicating with said sensor device, characterized in that said sensor device comprises a gas intake, a gas outlet, a first chamber, a second chamber, at least one membrane arranged to separate said chambers, at least one first gas sensor coupled to said first chamber and at least one second gas sensor coupled to said second chamber.

Not covered by the claims is a computer program product stored on a data carrier **SERVER.** Said computer program product comprises commands that perform a method according to the invention when operated in a system for controlling the transfer of a fuel present in a first location to a second location.

## BRIEF DESCRIPTION OF THE DRAWINGS

Fig. 1 is a schematic view of the mode of operation of the apparatus according to the invention.

Fig. 2 is an alternative view of the schematic mode of operation shown in Fig. 1.

Fig. 3 is an alternative view of the schematic mode of operation shown in Fig. 1.

Fig. 4 is a view of the apparatus according to the invention connected to a fuel dispenser nozzle.

Fig. 5 is a view of an alternative arrangement of Fig. 4.

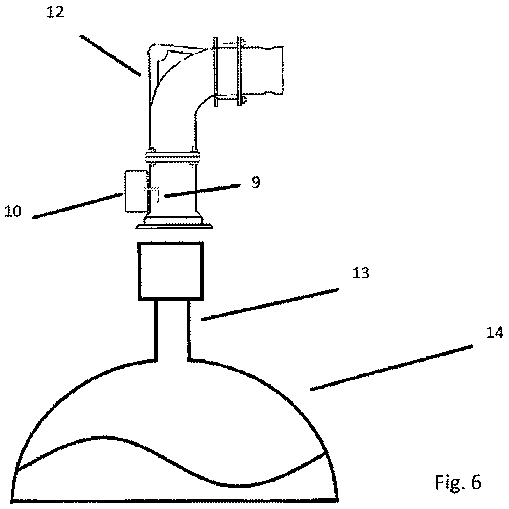

Fig. 6 is a view of the connecting conduit and the apparatus according to the invention while filling the fuel tank.

Fig. 7 is a view of an alternative arrangement of Fig. 6.

Fig. 8 is a symbolic cross-sectional side view of the apparatus with a membrane.

Fig. 9 is a symbolic cross-sectional side view of the apparatus with a membrane.

## REFERENCE NUMBERS OF THE ELEMENTS SHOWN IN THE FIGURES

1    Gas sensor

2    Controller
3    State sensor
4    Communication interface
5    Automation computer
6    Cloud server
7    Fuel dispenser
8    Dispenser nozzle
9    Suction tube
10   Main device
11   Sensor device
12   Filling elbow
13   Tank filling line
14   Fuel tank
15   Valve
16   Elbow gap
17   *First gas sensor*
18   Second gas sensor
19   Gas intake
20   Gas outlet
21   Membrane
22   Pore
23   Diesel molecules
24   Gasoline molecules
25   First chamber
26   Second chamber

## DETAILED DESCRIPTION OF THE INVENTION

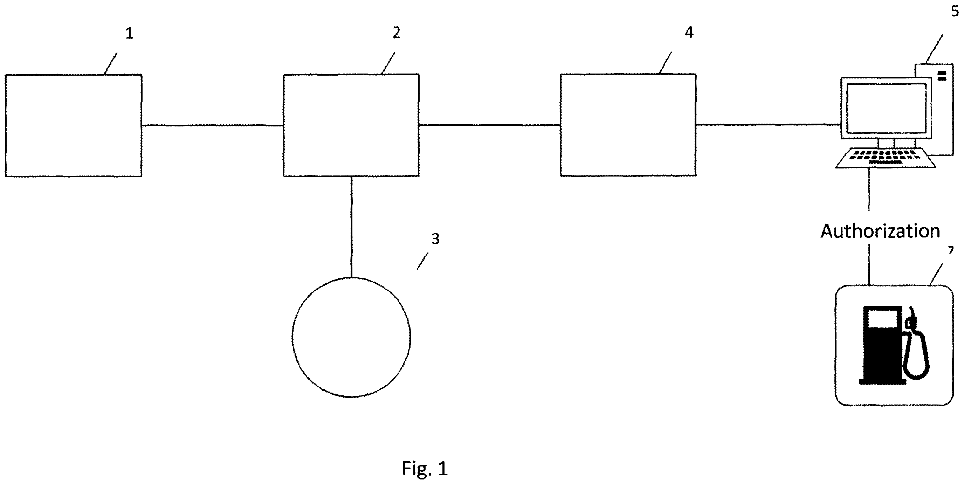

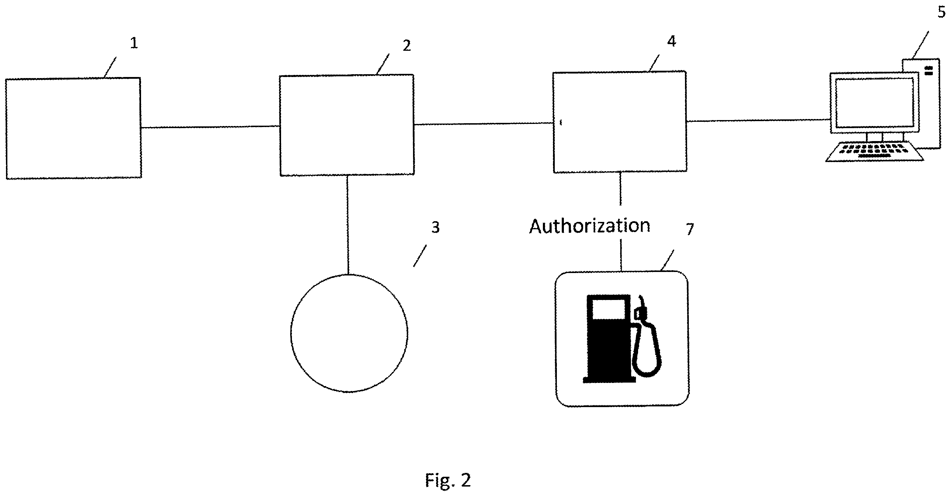

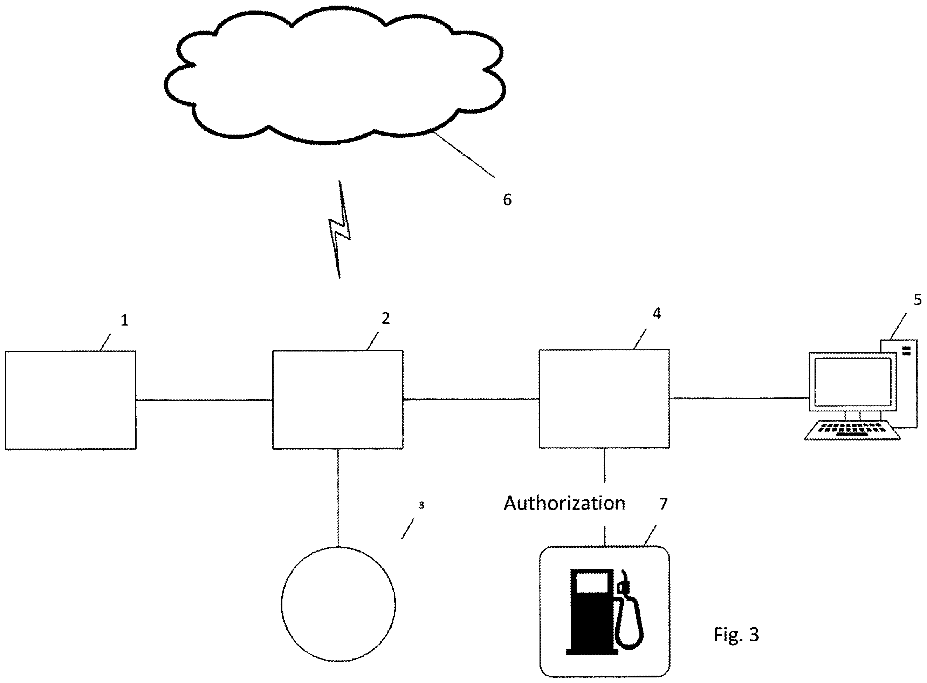

According to an embodiment of the invention, the first location may be a fuel dispenser (7) and the second location may be the fuel tank of a land vehicle. In a typical gas station, it is prior information as to which type of fuel is supplied from a particular fuel dispenser (7). Such information may be kept in a controller memory inside the main device which shall be described below or it may be stored in an automation computer (5) at the gas station as shown in Fig. 1 or in a memory connected to the fuel dispenser central processor as shown in Fig. 2 or a remote computer such as a cloud server (6) as shown in Fig. 3.

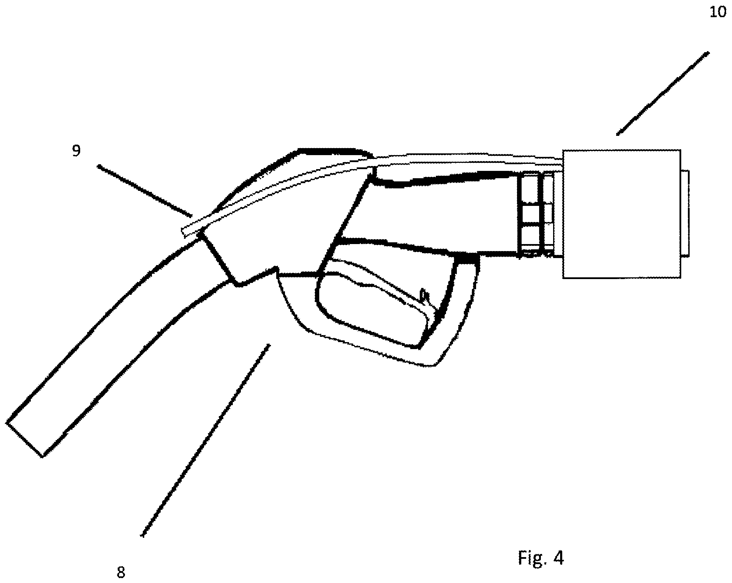

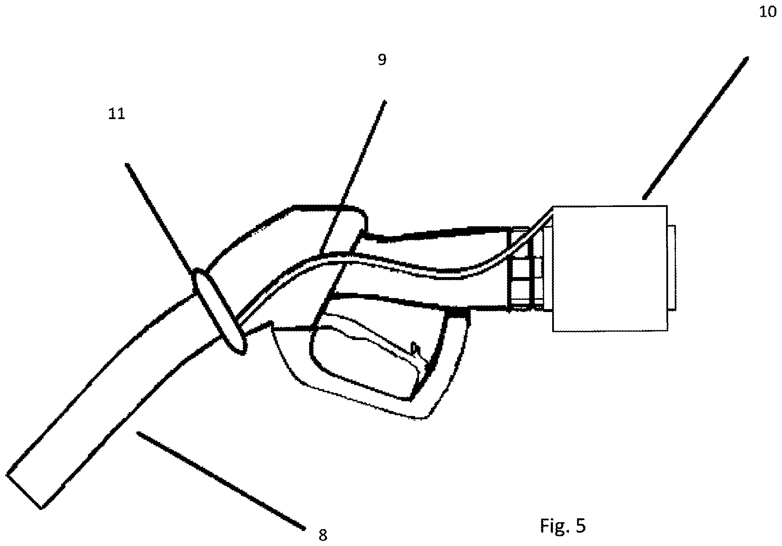

In such an arrangement, a main device (10) which is preferably detachably connected to the dispenser nozzle (8) as shown in Fig. 4 or 5, may be used for determining the fuel type in the fuel tank of the land vehicle. Said main device (10) basically comprises a sensor device (11) comprising a gas sensor (1) and a state sensor (3) as well as a controller (2) that can communicate with it. The sensor device is preferably connected to a suction tube (9) extending towards the end of the dispenser nozzle (8). The main device (10) may further comprise a fan for transmitting fuel vapor to the sensor device and a battery for operating same.

The gas sensor (1) may be a known VOC sensor or MEMS metal oxide sensor or air quality sensor or MEMS sensor or a combination thereof. When a plurality of sensors are used, they may be combined by a known method such as sensor fusion technique for generating more accurate results in the determination of gas type.

The controller (2) may at least be a microprocessor-based electronic unit having a memory unit, such as a microcontroller. In the controller memory, a predetermined data set is recorded as to the extent of volatility the gaseous form of each fuel type has under certain temperature and/or pressure conditions. Further, data as to what extent and at which rate the vapor of each fuel type permeates the membrane is also recorded. As shown in Fig. 1 to 3, a state sensor (3) is connected to the controller (2). Said state sensor (3) is a sensor known in the art that can detect the status characteristics of the gaseous form of the fuel in terms of temperature and/or pressure.

According to such an embodiment, in practice, after the land vehicle arrives at the gas station for refill, it stops next to a dispenser (7) having a fixed fuel type such as gasoline or diesel fuel and requests fuel of desired amount from the dispenser attendant. The dispenser attendant places the dispenser nozzle (8) into the tank inlet of the vehicle. Since the fuel in the vehicle tank is volatile, the gaseous form (or fuel vapor form) thereof is provided to the sensor device preferably through a suction tube (9) that extends towards the fuel tank of the vehicle wherein the density of the gas permeating or not permeating the membrane is detected by the gas sensor (1) whereas the temperature and/or pressure is detected by the status sensor. This data is compared with the corresponding data pre-recorded in the controller memory and classified. In this way, the type of the fuel present in the vehicle tank is detected. In this context, "corresponding data" means a combination of the data detected from the status sensor and the gas sensor at that moment.

With regard to the prevention of faulty fuel transfer, in practice, the most significant differentiation is between gasoline and diesel fuel. Gasoline molecules have 4 to 12 carbon atoms whereas diesel fuel molecules have 10 to 15 carbon atoms. Therefore, the molecular weight of diesel fuel is "heavier" compared to that of gasoline whereas its "volatility" under the same temperature and/or pressure conditions is lower. However, it may be possible for both fuel types to show identical or similar volatility under different temperature and/or pressure values.

Specific to gasoline and diesel fuel, the density of the gaseous forms of these two types of fuel under any identical temperature and/or pressure is different and this data is previously known. Therefore, while determining fuel type, if the density of diesel fuel vapor is A and that of gasoline is B under identical temperature and/or pressure, a threshold value can be determined via the mathematical average of A and B. Among the sampled gases, those which do not exceed this threshold value may be classified as diesel fuel and those which do as gasoline.

If the data received from the state sensor (3) does not match with the data set pre-recorded in the memory, the data detected will be estimated from the known data set. For achieving this, the k-Nearest Neighbor and/or Look-up table methods known in the art may be used for variance analysis, thereby classifying the fuel type.

Since the fuel type in the fuel dispenser is previously known and the fuel type in the vehicle tank has been determined, it will now be decided as to whether fuel transfer from the fuel dispenser (7) to the vehicle tank will be performed or not. This decision process may be conducted in terms of various systems or vehicles. For example, as shown in Fig. 1, such decision may be made in the automation computer (5) at the gas station or in a central processor unit in the fuel dispenser (7) as shown in Fig. 2 or a remote computer/cloud server (6) as shown in Fig. 3. The communication between the controller (2) and the decision unit may be enabled by any method known in the art, such as wired or wireless radio frequency based communication (4) (RF-based: Wi-fi, Bluetooth, Zigbee etc.) or a suitable communication method such as LoraWAN, 6LowPAN or LPWAN. As a result, if the fuel types in the fuel dispenser (7) and the vehicle tank are different, then fuel transfer from the dispenser to the vehicle will not be permitted, otherwise it will. This permission may be provided by communicating with the dispenser.

The sensor device may be arranged inside the main device (10) or, as shown in Fig. 5, in connection with the main device (10) but away from it and preferably closer to the fuel source the type of which will be determined (e.g. in a location near the end portion of the dispenser nozzle).

According to an embodiment of the invention, the fuel type to be supplied from the fuel dispenser may not be previously known but the fuel type in the tank of the land vehicle may be previously known. In such an embodiment, through for example a unique identifier (e.g. plate number) of each land vehicle, the fuel type used may have been previously identified on a database. In this case, the main device (10) will be arranged on the fuel dispenser (7) and the gaseous form of the fuel will be determined in the way described above and if it is different from the fuel type used by the land vehicle, fuel transfer will not be permitted, otherwise it will.

According to another embodiment of the invention, the fuel to be supplied from the fuel dispenser and the fuel type available in the tank of the land vehicle may not be previously known. In such an embodiment, a main device (10) will be arranged in the fuel dispenser (7) and the gaseous form of the fuel will be determined first as described above. Similarly, another main device will be arranged on the fuel dispenser nozzle as described above and the determination will be made again as described above. Alternatively, through a single main device, fuel types may be determined and compared at two different sources. If the fuel type present in the dispenser is different from the fuel type used by the land vehicle, fuel transfer will not be permitted, otherwise it will.

In cases where there is a self-service terminal with no refilling attendant for the fuel dispenser, a similar procedure as described above may still be implemented. In such an embodiment, the vehicle driver will choose the fuel type to be delivered to the vehicle on the user panel of the dispenser and place the dispenser nozzle (8) into the tank inlet. The main device (10) will detect the fuel type in the vehicle tank as described above and if there is no match with the fuel type the user has chosen on the dispenser, the dispenser will not permit fuel transfer to the vehicle, otherwise it will. When fuel transfer is not permitted, some alerts signals, such as audio or visual signals, may be generated.

According to an embodiment of the invention, the first location may be a fuel truck and the second location may typically be a fuel tank which may be buried underground at the station (14). In such a case, the fuel type in the fuel truck is generally known beforehand. Such information may be kept in various forms of data memory. For example, the fuel truck may involve such a data memory.

In the fuel transfer from a fuel truck to a fuel tank (14), filling elbows (12) coupled to various locking means are typically used. The main device (10) according to the invention is externally connected to the filling elbow (12) preferably detachably and in a way positioned near the tank refilling line (13). Again, in this embodiment, a suction tube (9) may be arranged that will transmit the gaseous fuel form to be sampled to the sensor device within the main device (10). The fuel gas sample taken from the suction tube (9) will be detected by the sensor device as described above and compared with the data set prerecorded in the controller memory or a mathematical modeling will be made, and the fuel type will be classified. If the fuel type present in the fuel truck is different from the fuel type in the tank, fuel transfer will not be permitted, otherwise it will. This permission may be provided for example by a solenoid valve at the fuel outlet of the tank keeping its closed position.

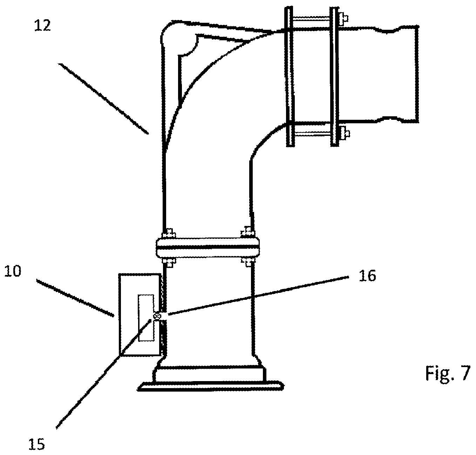

Fig. 7 shows an alternative embodiment according to which the main device (10) is externally connected to the filling elbow (12), in this embodiment however, the filling elbow (12) may optionally be in fuel vapor communication with the main device (10) through a gap (16). Such communication may be provided by an optional valve (15).

When the filling elbow (12) is placed on the filling cover of the tank, the valve (15) provides the transmission of the fuel vapor in gaseous form moving within the tank to the sensor device in the main device (10). On the other hand, when the fuel transfer is permitted, the valve (15) prevents the liquid fuel passing through the filling elbow (12) to leak into the main device (10).

The apparatus according to the invention may also be configured as a component of a fuel vapor recovery system. Such systems are known in the art and may generally be used in a fuel dispenser or fuel transfer between the fuel truck and the gas station tank. In order to recover the volatile fuel vapor at the second location where the fuel transfer will be made, a fuel vapor recovery system basically comprises a vacuum pump sucking the vapor, a pressurized or non-pressurized condensation chamber for converting the suctioned vapor into liquid form and lines providing transport among the components. The apparatus according to the invention may be arranged on any part of the fuel vapor recovery system, such as the condensation chamber, the line, or in connection with an element that provides a link to the line.

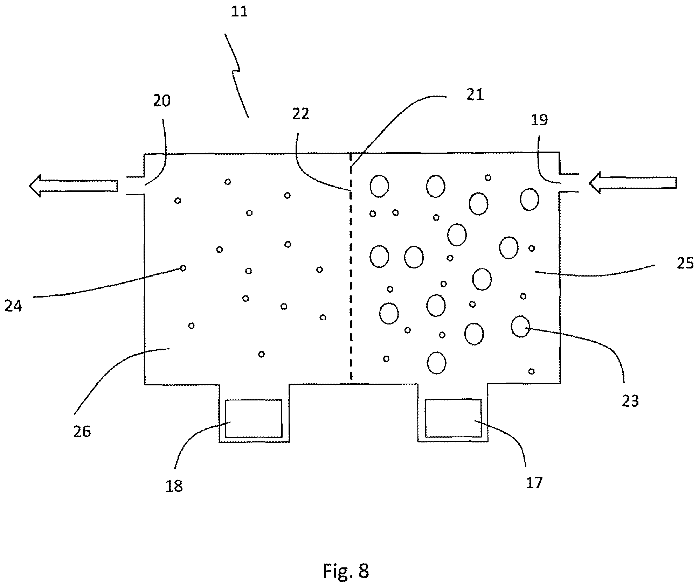

According to an embodiment of the invention, the fuel transfer control may be provided by a membraned sensor device. Fig. 8 shows an example for such a sensor device. The sensor device (11) defines a structure in substantially closed form containing a gas intake (19) and a gas outlet (20) preferably opposing each other. Inside said sensor device (11), there is an empty chamber divided into two sections through a membrane (21) to define a first chamber (25) and a second chamber (26).

The gas intake (19) is configured at the side of the first chamber (25) and the gas outlet (20) is configured at the side of the second chamber (26). The configuration of the membrane (21) comprises a frame which preferably comprises micro-scale pores (22) of 0.10-0.50 $\mu$m and one or more polymer layers accumulated thereon. As the microporous frame, various materials may be used such as polysulfone (PS), PI, polyetherimide (PEI), polyvinylidene fluoride (PVDF) or sulfonated tetrafluoroethylene fluoropolymer-copolymer (Nafion®). As the polymeric layer, various materials may be used such as polyether block amide (PEBA), polyethylene glycol (PEG), polyimide (PI), polyurethane (PU) or polydimethylsiloxane (PDMS). For enhancing the mechanical resistance and selectivity of the membrane, cross-linking within the polymeric layer or interlayer cross-linking may be applied. Cross-linking may be enabled through agents such as maleic anhydride, glutaraldehyde, etc. and also by heat treatment. The selectivity and gas permeation rates of the membrane may be adjusted by using inorganic nano-fillers on polymer layers, such as graphene oxide or carbon nanotubes.

The polymers in the quantity determined for the production of the membrane according to the invention may be dissolved by using appropriate solvents such as isopropanol/n-propanol, n-butanol or hexane. If it is aimed to use nano-fillers, then the selected inorganic material may be dispersed in this solution. The polymer-inorganic material mixture may be cast by using a membrane casting apparatus in order to prepare a microporous frame structure of the desired thickness.

The accumulation process is performed on the selected microporous frame. Parameters such as the duration of the process, selection of the solvent medium, need for a catalyst and the solvent removal step may be determined depending on the frame and the polymer.

The synthesized membranes may be subjected to various functionality tests such as mechanical and thermal resistance and gas permeability, thereby determining the selectivity values.

The size of the pores (22) of the membrane (21) will permit fuel gas molecules of certain molecular size to permeate whereas larger molecules will not be permitted. As shown in Fig. 8, when diesel gas enters into the gas intake, since diesel molecules (23) will not permeate through the membrane pores (22), they will accumulate in the first chamber (25). As is known, diesel gas may not always wholly comprise diesel molecules and may also have smaller molecules, i.e. molecules of the size of gasoline molecules (21) even at small rates, a portion of which will permeate through the membrane pores (22) and accumulate in the second chamber (26). Accordingly, the membrane (21) will separate gas molecules of different sizes so that they can be kept in the first chamber (25) and the second chamber (26), respectively.

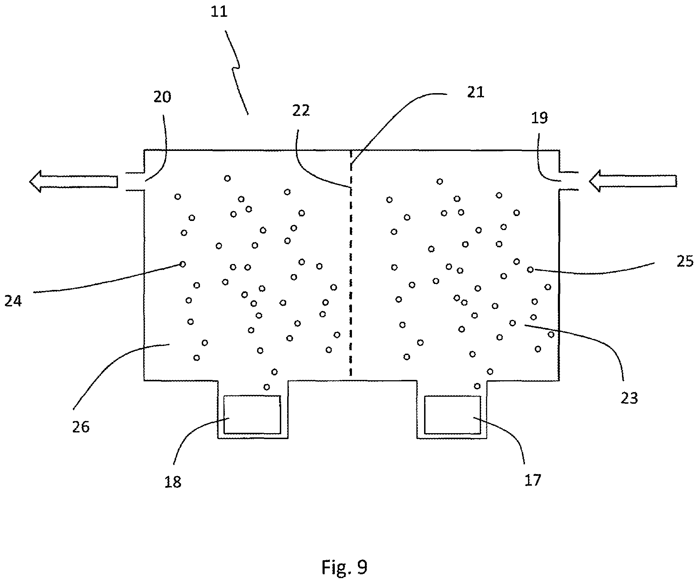

The first chamber (25) is provided with a first gas sensor (17) and the second chamber (26) is provided with a second gas sensor (18). The first gas sensor (17) and the second gas sensor (18) may for example be a known VOC sensor or MEMS metal oxide sensor or air quality sensor or MEMS sensor. Said sensors (17, 18) communicate with a control unit. In practice, the signal received from the first gas sensor (17) (the signal related to the gas density level) and the signal received from the second gas sensor (18) (the signal related to the gas density level) are compared and interpreted by the control unit. If the signal received from the first gas sensor (17) is different from the signal received from the second gas sensor (18) on the basis of a predetermined tolerance range and if the rate between said signals is within a tolerance range determined for diesel vapor, then it may be decided that the gas mixture entering into the sensor device (11) is a large-molecule gas mixture formed by the vaporization of diesel. For example, as shown in Fig. 9, if there is a specific rate between the signal received from the first gas sensor (17) and the signal received from the second gas sensor (18) based on the predetermined tolerance range for gasoline vapor, then it may be decided that the gas mixture entering into the sensor device (11) is a gas mixture formed by the vaporization of gasoline.

According to an embodiment of the invention, a suction fan may be arranged at the gas outlet (20) side, thereby enabling a more regular and rapid exit of the gas entering through the gas intake (19) from the sensor device (11) chamber.

According to an embodiment of the invention, a suction fan may be arranged at the gas intake (19) side, thereby enabling a more regular and rapid introduction of the gas through the gas intake (19) inside the sensor device (11) chamber.

According to an embodiment of the invention, the fan arranged at the gas intake (19) side may enable both a more regular and rapid introduction of the gas through the gas intake (19) inside the sensor device (11) chamber and also rapid discharge of the fuel vapor entering into the sensor device (11) chamber through the gas intake (19) again from the intake (19).

Claims

1. A method for controlling the transfer of a known-type or an unknown-type fuel from a first location to a second location with a known-type or an unknown type fuel, comprising the following steps of:

   a. determining the unknown fuel type in at least one of said locations,
   b. comparing the fuel type in the second location with the fuel type in the first location,
   c. permitting fuel transfer, in case the fuel type in the first location matches with the fuel type in the second location, and preventing the transfer otherwise,

   wherein the determination in the step a comprises measuring the density and temperature, or measuring the density and pressure of a gaseous form of the unknown fuel in the respective location(s), and comparing the measured values with a data set comprising predetermined reference values or making a decision through a mathematical modelling.

2. The method according to Claim 1, wherein the first location is a fuel dispenser (7) and the second location is the fuel tank of a land vehicle.

3. The method according to Claim 1, wherein the first location is the tank of a fuel truck and the second location is the tank (14) of a gas station.

4. The method according to Claim 1, wherein the fuel either in the first location or in the second location is either gasoline or diesel.

5. The method according to Claim 1, wherein the density of the gaseous form of the fuel is measured by a gas sensor (1), the temperature or pressure is measured by a state sensor (3), and the data received from the sensors are processed by a controller (2) generating a command signal by comparing the data with a data set comprising predetermined reference values or through mathematical modelling.

6. The method according to Claim 1, wherein if the data obtained from temperature or pressure measurement do not match with the data set comprising predetermined reference values or as a result of a mathematical modelling, the obtained data are estimated from a pre-prepared data set.

7. An apparatus for controlling the transfer of a type of fuel from a first location to a second location, comprising a sensor device (11) for detecting the type of the fuel in one of said locations and a controller (2) communicating with the sensor device, wherein the controller (2) is configured to compare the data received from said sensor device with a data set comprising predetermined reference values or to conduct a mathematical modelling, **characterized in that** the sensor device (11) comprises a gas sensor (1) for detecting the density of the gaseous form of the fuel in a corresponding location and at least one gaseous state sensor for detecting the temperature or pressure of the gaseous form of the fuel in a corresponding location.

8. The apparatus according to claim 7, **characterized in that** said sensor device (11) comprises a gas intake (19), a gas outlet (20), a first chamber (25), a second chamber (26), at least a membrane (21) arranged to separate said chambers, at least one first gas sensor (17) communicating with said first chamber (25) and at least one second gas sensor (18) communicating with said second chamber (26).

9. The apparatus according to Claim 8, wherein the membrane (21) comprises a microporous frame and one or more polymer layers accumulated thereon.

10. The apparatus according to Claim 9, wherein the microporous frame is selected from the group consisting of polysulfone (PS), PI, polyetherimide (PEI), polyvinylidene fluoride (PVDF) and sulfonated tetrafluoroethylene fluoropolymer- copolymer (Nafion®), and the polymer is selected from the group consisting of polyether block amide (PEBA), polyethylene glycol (PEG), polyimide (PI), polyurethane (PU) and polydimethylsiloxane (PDMS).

11. The apparatus according to Claim 10, wherein said polymer comprises cross-linking by an agent selected from the group consisting of maleic anhydride and glutaraldehyde.

12. The apparatus according to Claim 10, wherein said polymer comprises an inorganic nano-filler selected from the group consisting of graphene oxide and carbon nanotubes.

13. The apparatus according to Claim 8, wherein said at least one first gas sensor (17) and at least one second gas sensor (18) are selected from the group consisting of a VOC sensor, MEMS metal oxide sensor, air quality sensor and MEMS sensor.

14. The apparatus according to Claim 8, wherein the apparatus comprises at least one fan.

15. The apparatus according to Claim 14, wherein the apparatus comprises at least one suction fan provided at the side of said gas intake and/or at the side of said gas outlet.

**Patentansprüche**

1. Ein Verfahren zum Steuern des Transfers eines Kraftstoffs eines bekannten Typs oder eines unbekannten Typs von einem ersten Ort zu einem zweiten Ort mit einem Kraftstoff eines bekannten Typs oder eines unbekannten Typs, das die folgenden Schritte beinhaltet:

   a. Bestimmen des unbekannten Kraftstofftyps an mindestens einem der Orte,
   b. Vergleichen des Kraftstofftyps an dem zweiten Ort mit dem Kraftstofftyp an dem ersten Ort,
   c. Ermöglichen des Kraftstofftransfers in dem Fall, dass der Kraftstofftyp an dem ersten Ort mit dem Kraftstofftyp an dem zweiten Ort übereinstimmt, und ansonsten Verhindern des Transfers,

   wobei die Bestimmung in dem Schritt a das Messen der Dichte und der Temperatur oder das Messen der Dichte und des Drucks einer gasförmigen Form des unbekannten Kraftstoffs an dem/den jeweiligen Ort(en) und das Vergleichen der gemessenen Werte mit einem Datensatz, der vorgegebene Bezugswerte beinhaltet, oder das Treffen einer Entscheidung mittels mathematischer Modellierung beinhaltet.

2. Verfahren gemäß Anspruch 1, wobei der erste Ort eine Zapfsäule (7) ist und der zweite Ort der Kraftstofftank eines Landfahrzeugs ist.

3. Verfahren gemäß Anspruch 1, wobei der erste Ort der Tank eines Tanklasters ist und der zweite Ort der Tank (14) einer Tankstelle ist.

4. Verfahren gemäß Anspruch 1, wobei der Kraftstoff entweder an dem ersten Ort oder an dem zweiten Ort entweder Benzin oder Diesel ist.

5. Verfahren gemäß Anspruch 1, wobei die Dichte der gasförmigen Form des Kraftstoffs durch einen Gassensor (1) gemessen wird, die Temperatur oder der Druck durch einen Zustandssensor (3) gemessen wird und die von den Sensoren empfangenen Daten durch eine Steuereinheit (2) verarbeitet werden, welche durch Vergleichen der Daten mit einem Datensatz, der vorgegebene Bezugswerte beinhaltet, oder mittels mathematischer Modellierung ein Befehlssignal erzeugt.

6. Verfahren gemäß Anspruch 1, wobei, wenn die aus der Temperatur- oder Druckmessung erhaltenen Daten nicht mit dem Datensatz, der die vorgegebenen Bezugswerte beinhaltet, übereinstimmen oder als Ergebnis einer mathematischen Modellierung, die erhaltenen Daten aus einem vorbereiteten Datensatz geschätzt werden.

7. Eine Vorrichtung zum Steuern des Transfers eines Typs von Kraftstoff von einem ersten Ort zu einem zweiten Ort, beinhaltend ein Sensorgerät (11) zum Detektieren des Typs des Kraftstoffs an einem der Orte und eine Steuereinheit (2), die mit dem Sensorgerät kommuniziert, wobei die Steuereinheit (2) konfiguriert ist, um die von dem Sensorgerät empfangenen Daten mit einem Datensatz, der vorgegebene Bezugswerte beinhaltet, zu vergleichen oder um eine mathematische Modellierung durchzuführen, **dadurch gekennzeichnet, dass** das Sensorgerät (11) einen Gassensor (1) zum Detektieren der Dichte der gasförmigen Form des Kraftstoffs an einem entsprechenden Ort und mindestens einen Gasform-Zustandssensor zum Detektieren der Temperatur oder des Drucks der gasförmigen Form des Kraftstoffs an einem entsprechenden Ort beinhaltet.

8. Vorrichtung gemäß Anspruch 7, **dadurch gekennzeichnet, dass** die Sensorvorrichtung (11) eine Gasaufnahme (19), einen Gasauslass (20), eine erste Kammer (25), eine zweite Kammer (26), mindestens eine Membran (21), die zum Trennen der Kammern eingerichtet ist, mindestens einen ersten Gassensor (17), der mit der ersten Kammer (25) kommuniziert, und mindestens einen zweiten Gassensor (18), der mit der zweiten Kammer (26) kommuniziert, beinhaltet.

9. Vorrichtung gemäß Anspruch 8, wobei die Membran (21) einen mikroporösen Rahmen und eine oder mehrere darauf abgelagerte Polymerschichten beinhaltet.

10. Vorrichtung gemäß Anspruch 9, wobei der mikroporöse Rahmen aus der Gruppe ausgewählt ist, die aus Folgendem besteht: Polysulfon (PS), PI, Polyetherimid (PEI), Polyvinylidenfluorid (PVDF) und sulfoniertem Tetrafluorethylenfluorpolymer-Copolymer (Nafion®), und das Polymer aus der Gruppe ausgewählt ist, die aus Folgendem besteht: Polyetherblockamid (PEBA), Polyethylenglycol (PEG), Polyimid (PI), Polyurethan (PU) und Polydimethylsiloxan (PDMS).

11. Vorrichtung gemäß Anspruch 10, wobei das Polymer Vernetzung durch ein Mittel, ausgewählt aus der Gruppe, bestehend aus Maleinsäureanhydrid und Glutaraldehyd, beinhaltet.

12. Vorrichtung gemäß Anspruch 10, wobei das Polymer einen anorganischen Nanofüllstoff, ausgewählt aus der Gruppe, bestehend aus Graphenoxid und Kohlenstoffnanoröhrchen, beinhaltet.

13. Vorrichtung gemäß Anspruch 8, wobei der mindestens eine erste Gassensor (17) und mindestens eine zweite Gassensor (18) aus der Gruppe ausgewählt sind, die aus Folgendem besteht: einem VOC-Sensor, einem MEMS-Metalloxidsensor, einem Luftqualitätssensor und einem MEMS-Sensor.

14. Vorrichtung gemäß Anspruch 8, wobei die Vorrichtung mindestens ein Gebläse beinhaltet.

15. Vorrichtung gemäß Anspruch 14, wobei die Vorrichtung mindestens ein Sauggebläse beinhaltet, das an der Seite der Gasaufnahme und/oder an der Seite des Gasauslasses bereitgestellt ist.


**Revendications**

1. Un procédé destiné à commander le transfert d'un type connu ou d'un type inconnu de carburant d'un premier emplacement à un deuxième emplacement avec un type connu ou un type inconnu de carburant, comprenant les étapes suivantes consistant à :

   a. déterminer le type de carburant inconnu dans au moins un desdits emplacements,
   b. comparer le type de carburant dans le deuxième emplacement au type de carburant dans le premier emplacement,
   c. permettre un transfert de carburant, dans le cas où le type de carburant dans le premier emplacement coïncide avec le type de carburant dans le deuxième emplacement, et sinon empêcher le transfert,

   dans lequel la détermination à l'étape a comprend le fait de mesurer la masse volumique et la température, ou de mesurer la masse volumique et la pression d'une forme gazeuse du carburant inconnu dans l'emplacement ou les emplacements respectif(s), et de comparer les valeurs mesurées à un ensemble de données comprenant des valeurs de référence prédéterminées ou de prendre une décision par le biais d'une modélisation mathématique.

2. Le procédé selon la revendication 1, dans lequel le premier emplacement est un distributeur de carburant (7) et le deuxième emplacement est le réservoir de carburant d'un véhicule terrestre.

3. Le procédé selon la revendication 1, dans lequel le premier emplacement est le réservoir d'un camion-citerne à carburant et le deuxième emplacement est le réservoir (14) d'une station-service.

4. Le procédé selon la revendication 1, dans lequel le carburant soit dans le premier emplacement, soit dans le deuxième emplacement est soit de l'essence, soit du diesel.

5. Le procédé selon la revendication 1, dans lequel la masse volumique de la forme gazeuse du carburant est mesurée par un capteur de gaz (1), la température ou la pression est mesurée par un capteur d'état (3), et les données reçues en provenance des capteurs sont traitées par un organe de commande (2) générant un signal d'instruction par comparaison des données à un ensemble de données comprenant des valeurs de référence prédéterminées ou par le biais d'une modélisation mathématique.

6. Le procédé selon la revendication 1, dans lequel si les données obtenues à partir d'une mesure de température ou de pression ne coïncident pas avec l'ensemble de données comprenant des valeurs de référence prédéterminées ou en résultat d'une modélisation mathématique, les données obtenues sont estimées à partir d'un ensemble de données prépréparé.

7. Un appareil destiné à commander le transfert d'un type de carburant d'un premier emplacement à un deuxième emplacement, comprenant un dispositif capteur (11) destiné à détecter le type de carburant dans un desdits emplacements et un organe de commande (2) communiquant avec le dispositif capteur, dans lequel l'organe de commande (2) est configuré pour comparer les données reçues en provenance dudit dispositif capteur à un ensemble de données comprenant des valeurs de référence prédéterminées ou pour exécuter une modélisation mathématique, **caractérisé en ce que** le dispositif capteur (11) comprend un capteur de gaz (1) destiné à détecter la masse volumique de la forme gazeuse du carburant dans un emplacement correspondant et au moins un capteur d'état gazeux destiné à détecter la température ou la pression de la forme gazeuse du carburant dans un emplacement correspondant.

8. L'appareil selon la revendication 7, **caractérisé en ce que** ledit dispositif capteur (11) comprend une admission de gaz (19), une évacuation de gaz (20), une première chambre (25), une deuxième chambre (26), au moins une membrane (21) agencée pour séparer lesdites chambres, au moins un premier capteur de gaz (17) communiquant avec ladite première chambre (25) et au moins un deuxième capteur de gaz (18) communiquant avec ladite deuxième chambre (26).

9. L'appareil selon la revendication 8, dans lequel la membrane (21) comprend un cadre microporeux et une ou plusieurs couches de polymère accumulées sur celui-ci.

10. L'appareil selon la revendication 9, dans lequel le cadre microporeux est sélectionné dans le groupe constitué de polysulfone (PS), de PI, de polyétherimide (PEI), de polyfluorure de vinylidène (PVDF) et d'un copolymère fluoropolymère tétrafluoroéthylène sulfoné (Nafion®), et le polymère est sélectionné dans le groupe constitué de polyéther bloc amide (PEBA), de polyéthylène glycol (PEG), de polyimide (PI), de polyuréthane (PU) et de polydiméthylsiloxane (PDMS).

11. L'appareil selon la revendication 10, dans lequel ledit polymère comprend une réticulation par un agent sélectionné dans le groupe constitué d'anhydride maléique et de glutaraldéhyde.

12. L'appareil selon la revendication 10, dans lequel ledit polymère comprend une nanocharge inorganique sélectionnée dans le groupe constitué de nanotubes de carbone et d'oxyde de graphène.

13. L'appareil selon la revendication 8, dans lequel lesdits au moins un premier capteur de gaz (17) et au moins un deuxième capteur de gaz (18) sont sélectionnés dans le groupe constitué d'un capteur de COV, d'un capteur d'oxyde métallique MEMS, d'un capteur de qualité de l'air et d'un capteur MEMS.

14. L'appareil selon la revendication 8, l'appareil comprenant au moins un ventilateur.

15. L'appareil selon la revendication 14, l'appareil comprenant au moins un ventilateur aspirant prévu au niveau du côté de ladite admission de gaz et/ou au niveau du côté de ladite évacuation de gaz.

Fig. 1

EP 3 776 503 B1

Authorization

Fig. 2

Fig. 3

Fig. 4

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4838323 A
- US 6341629 A
- US 5209275 A
- DE 102011112417 A1
- EP 0566345 A1